# EUROPÄISCHE PATENTSCHRIFT

(11) **EP 1 133 290 B1**
(45) Veröffentlichungstag und Bekanntmachung des Hinweises auf die Patenterteilung: **12.03.2003**
(21) Anmeldenummer: 99955589.9
(22) Anmeldetag: 22.11.1999
(51) Int. Cl.: A61K 31/27, A61P 25/28, A61P 25/30

(54) **VERWENDUNG VON ACETYLCHOLINESTERASE-INHIBITOREN ZUR BEHANDLUNG VON DELIRIEN**
USE OF INHIBITORS OF ACETYLCHOLINESTERASE FOR THE TREATMENT OF DELIRIUMS
UTILISATION D'INHIBITEURS D'ACETYLCHOLINESTERASE POUR LE TRAITEMENT DES DELIRIUMS

(30) Priorität: 27.11.1998 AT 199598
(43) Veröffentlichungstag der Anmeldung: 19.09.2001
(73) Patentinhaber: Sanochemia Pharmazeutika Aktiengesellschaft, 1090 Wien (AT)
(72) Erfinder: FISCHER, Peter, 1170 Wien (AT)
(74) Vertreter: Beer, Manfred, Dipl.-Ing.
(86) Internationale Anmeldenummer: AT9900284
(87) Internationale Veröffentlichungsnummer: WO00032185

(56) Entgegenhaltungen:
- EP-A- 0 821 957
- WO-A-98/17214
- WO-A-98/31356
- LEROI I. ET AL: "The care of patients with dementia." REVIEWS IN CLINICAL GERONTOLOGY, (1999) 9/3 (235-255). , XP000878802
- GALASKO D.: "A clinical approach to dementia with Lewy bodies." NEUROLOGIST, (1999) 5/5 (247-257). , XP000878694
- WILCOCK: "tacrine for senile dementia..." LANCET, Nr. 344, 1994, Seite 544 XP000876988

## Beschreibung

Die Erfindung betrifft die Verwendung von Acetylcholinesterase-Hemmern zur Herstellung einer Präparation zur Behandlung von Deliren, die nicht durch anticholinerge Intoxikation oder Degenerierung des cholinergen Systems bedingt sind.

Acetylcholin ((2-Acetoxyethyl)-trimethylammonium-hydroxid) ist der Neurotransmitter der cholinergen Synapsen. Cholinerge Synapsen finden sich in sämtlichen Bahnen des parasympathischen Nervensystems, an den präganglionären Sympathikusfasern, den peripheren motorischen Neuronen und in praktisch allen Regionen des zentralen Nervensystems (an allen cholinergen Synapsen des zentralen Nervensystems).

Acetylcholin wird im Axon in der Nähe der präsynaptischen Membran durch das Enzym Cholin-O-Acetyltransferase durch Acetylierung von Cholin gebildet und in den synaptischen Vesikeln gespeichert. Bei Erregung der präsynaptischen Nervenzelle strömen Ca-Ionen ein und bewirken die Freisetzung von Acetylcholin in den synaptischen Spalt. An der postsynaptischen Membran bindet Acetylcholin an spezielle Rezeptoren ("Acetylcholinrezeptoren"), wodurch eine Signalübertragung an die postsynaptische Zelle erreicht wird. Postsynaptische Zellen können dabei z.B. eine weitere Nervenzelle, eine Muskelzelle oder eine Drüsenzelle sein. Ein Abbau des Acetylcholins in Cholin und Acetat durch das Enzym Acetylcholin-Esterase bewirkt die Beendigung des Erregungszustandes und die Wiederherstellung der Erregungsbereitschaft. Cholin kann anschließend von der präsynaptischen Zelle erneut aufgenommen und wiederverwertet werden.

Bei den Acetylcholin-Rezeptoren unterscheidet man prinzipiell zwei Typen, die aufgrund ihrer unterschiedlichen Reaktion auf die Agonisten Muscarin und Nicotin als nicotinische und muscarinische Acetylcholin-Rezeptoren bezeichnet werden.

Nicotinische Rezeptoren sind an den sympathischen und parasympathischen Ganglien, an den neuromuskulären Endplatten und auch im Gehirn lokalisiert. Ihr Vorkommen im Gehirn wird mit dem Lernen, der Wirkung des Nicotins auf die Lernfähigkeit und mit der Alzheimerschen Krankheit in Verbindung gebracht. Der nicotinische Acetylcholin-Rezeptor gilt als der bestuntersuchteste Rezeptor eines Neurotransmitters und besteht aus fünf Polypeptidketten (2 α-, jeweils eine β-, γ- und δ-Kette), die eine fünfeckige Pore in der Membran bilden. Diese Pore wirkt als Kationen-Kanal, der geöffnet wird, sobald Acetylcholin an beide α-Untereinheiten gebunden ist. Als Hemmstoffe dieses Ionenkanals wirken verschiedene Schlangengifte (z.B. α-Bungarotoxin, Kobratoxin), das Pfeilgift Curare ((+)-Tubocurarin), Lophotoxin sowie etliche quarternäre Ammoniumverbindungen, die anstelle von Acetylcholin binden können. Es gibt eine noch unbestimmte Zahl (mindestens jedoch 3) Subtypen des nicotinischen Acetylcholin-Rezeptors.

Muscarinische Rezeptoren bestehen aus einer einzigen Polypeptidkette und üben ihren Einfluß über G-Proteine und verschiedene Effektoren, wie Phospholipase C oder Adenylat-Cyclase aus. Es werden fünf Subtypen pharmakologisch definiert, wobei im zentralen Nervensystem hauptsächlich M₁- und M₄- und nur in geringen Mengen M₂- und M₃- und M₅-Rezeptoren vorkommen. Die exzitatorische Wirkung der M₁- und M₃-Rezeptoren und deren Lokalisation im Hippocampus lassen bei Aktivierung dieser Rezeptoren positive kognitive Effekte erwarten, während über den M₂-Rezeptor eine inhibitorische Wirkung erzielt wird und keine Besserung kognitiver Defizite erwartet werden können. Hingegen würden auch Antagonisten am M₂-Rezeptor das zentrale cholinerge Nervensystem aktivieren.

Arzneimittel, die eine mit Acetylcholin vergleichbare erregungsübertragende Wirkung haben, werden als Cholinergika oder Parasympathikomimetika bezeichnet und solche, die diesen Prozessen entgegenwirken, also z.B. die Acetylcholin-Rezeptoren hemmen, als Anticholinergika oder Parasympathikolytika.

Die Acetylcholin-Esterase besteht aus 4 identischen Untereinheiten und gehört zur Gruppe der Serin-Esterasen. Ein Mol dieses Enzyms hydrolisiert rund 25 000 Mol Acetylcholin pro Sekunde. Diese extrem hohe Umsatzgeschwindigkeit ist wesentlich für die rechtzeitige Beendigung der durch Acetylcholin übertragenen Nervenimpulse (in 0,1 ms ist alles im synaptischen Spalt vorhandene Acetylcholin hydrolysiert). Bekannte (reversible) Hemmstoffe der Acetylcholin-Esterase sind Demecariumbromid, Neostigmin, Pyridostigminbromid sowie Physostigmin und verwandte Carbaminsäureester. Eine irreversible Hemmung wird auch durch Diisopropylfluorophosphat, Parathion (=E605), Tetrastigmin oder durch Phosphor-organische Verbindungen (die z.T. als Kampfgase oder Schädlingsbekämpfungsmittel verwendet werden, z.B. Phosgen, Alkylphosphate, etc.) herbeigeführt.

Zu den Parasympathikomimetika zählen neben den Acetylcholin-Esterase-Hemmern auch Cholinester, wie (neben Acetylcholin) Bethanechol, Carbachol und Methacholin, und Alkaloide, wie Muscarin und Pilocarpin. Weiters wirken die Cholinesterasehemmer Physostigmin und Galanthamin aber auch z.B. (+)-2-Methylpiperidine als Kanal-Aktivatoren am nicotinischen Acetylcholinrezeptoren. Auch gibt es nicotinische (Nicotin, Cytisine, Lobeline, Anatoxin-a, Epibatidine, 2,4-Dimethyl-cinnamylidene anabaseine, 2,4-Dimethyloxybenzylidene anabaseine und ABT-418, das Isoxazol-Isostere von Nicotin) und muscarinische Rezeptor-Agonisten (RS86, Arecoline, Oxotremorine, AF102B, Azaspirodecanes, etc.).

Seit wenigen Jahren stehen zur Behandlung der Demenz vom Alzheimertyp Medikamente zur Verfügung, die dem zentralnervösen Mangel an Acetylcholin bei dieser Erkrankung entgegenwirken und so die Hirnleistung verbessern. In Österreich sind in dieser Indikation die Substanzen Galanthamin (Nivalin®), Tacrin (Cognex®), Donepezil (Aricept®) und Rivastigmin (Exelon®) zugelassen. Weitere zentrale Hemmer der Acetylcholin-Esterase sind weltweit in Entwicklung (z.B. Metrifonate®, Bayer Corporation; Synapton®=Physostigmin slow release, Forest Laboratories; Galantamine-Reminyl, Janssen Pharmaceutica; Eptastigmine, Mediolanum; Velnacrine, Hoechst-Roussel; Suronacrine, Hoechst-Roussel; Huperzine A, Chinese Academy of Science; NX-066, Astra Arcus; KA-672, Schwabe; etc.). Weiters werden zur Zeit eine Reihe an Agonisten des cholinergen Systems, insbesondere M1-Agonisten, in ihrer Wirkung bei Demenz vom Alzheimertyp klinisch oder vorklinisch getestet (z.B. Memric®, SmithKline Beecham; Talsaclidine®, Boehringer Ingelheim Pharmaceuticals; etc). Der parenteral zu verabreichende Hemmer der Acetylcholin-Esterase Physostigmin ist in praktisch allen Ländern der Welt als Antidot bei Vergiftungen mit anticholinergen Substanzen (Atropin, Antidepressiva, Neuroleptika, ..) in Ampullenform seit Jahrzehnten zugelassen. In Österreich als Anticholium-Ampullen mit Indikation "Antidot bei Vergiftungen, zentral anticholinerges Syndrom".

Neben den Demenzen, also den chronischen organisch bedingten psychischen Störungen, gibt es die akuten organischen Psychosen, die im Laufe der Geschichte der Psychiatrie sehr unterschiedliche Namen trugen. Heute sprechen beide internationalen Diagnosesysteme psychiatrischer Krankheiten vom akuten Verwirrtheitszustand und verwenden den Begriff Delir synonym. Die Inzidenz der akuten Verwirrtheitszustände ist bedeutend größer als die der Demenzen. Das Auftreten eines akuten Verwirrtheitszustands ist Folge akuter Beeinträchtigung der Funktion des zentralen Nervensystems und kann in jedem Alter auch ohne strukturelle Schädigung des Hirngewebes auftreten. Allerdings ist hohes Lebensalter ein Risikofaktor für das Auftreten akuter Verwirrtheitszustände, jedoch leiden nur etwa 25 % der Patienten mit akutem Verwirrtheitszustand an internen Krankenhausabteilungen auch an einer Demenzerkrankung (Erkinjuntti et al (1986) Arch Int Med 146:1923-1926). Eine Vorschädigung des Gehirns wie sie beim dementen Patienten besteht, erhöht die Wahrscheinlichkeit bei akuter Funktionsstörung ein Delir zu entwickeln. Die Ursachen eines akuten Verwirrtheitszustands sind überaus zahlreich.

Die häufigsten akuten Verwirrtheitszustände treten heute an internen und chirurgischen Stationen in Folge interner Krankheiten bzw. nach Traumen oder nach Operationen auf. Weiters treten akute Verwirrtheitszustände auch nach nicht anticholinergen Intoxikationen (z.B. durch Lithium, siehe geschilderter Einzelfall) oder bei Substanzentzug (Alkoholentzug, Tranquilizerentzug) auf. Akute Verwirrtheitszustände verlängern die Krankenhausaufenthalte, erhöhen die Komplikationsraten der zugrundeliegenden Erkrankungen und erhöhen die Mortalität dieser Erkrankungen. Ihre Behandlung erfolgt derzeit einerseits durch den Versuch die Ursache zu beseitigen (z.B. Erhöhung des Blutdrucks, Antibiotikagabe bei Pneumonie, ..) und bei Selbstgefährdung oder Fremdgefährdung der verwirrten und oft erregten Patienten durch Gabe hochpotenter Neuroleptika, die ihrerseits nebenwirkungsreich sind.

Generell wird unter einem anticholinergen Delir ein Delir verstanden, das unter Gabe von anticholinerg wirkenden Substanzen auftritt. Als nicht-anticholinerge Delirien werden hingegen Delirien bezeichnet, die auftreten, ohne daß anticholinerg wirkende Substanzen im Vorfeld (also in der Regel 48 - 72 h vorher) verabreicht wurden.

Bei der Behandlung von Delirien steht daher gegenwärtig vor allem die Therapie der organischen Ursache im Vordergrund der Behandlung. Die Ermittlung der Ursache, auf die dann die (medikamentöse) Delir-Behandlung abgestellt wird ist jedoch aufgrund der Vielzahl an möglichen Ursachen äußerst aufwendig und zeitraubend, so daß eine schnelle Behandlung in der Regel schwierig ist. In der Regel geht man zuerst immer von der Annahme eines medikamentös verursachten Delirs aus und setzt daher anfangs jede nicht vital induzierte und in jedem Fall jede anticholinerg wirkende Medikation, sowie Medikationen, die die Blut-Hirnschranke passierten, ab. Daneben muß bei einem heftig halluzinierenden und die nächtliche Ruhe anderer Mitpatienten störenden deliranten Patienten symptomatisch mit hochpotenten Neuroleptika (z.B. Haloperidol) behandelt werden. Eine solche neuroleptische Medikation des deliranten Patienten muß jeden Tag aufs neue bezüglich der Indikation und Dosis überprüft werden.

Andererseits weisen die akuten Verwirrtheitszustände aber eine höhere Inzidenz als selbst dementielle und depressive Syndrome bei alten Menschen auf (so werden bei 30 bis 50 % der Patienten über 70 Jahre zu irgendeinem Zeitpunkt während einer internen Aufnahme akute Verwirrtheitszustände beobachtet), so daß hier ein großer Bedarf an einer schnellen, universell anwendbaren Behandlung besteht.

Insgesamt wird geschätzt, daß etwa 10 % der intern oder chirurgisch hospitalisierten Patienten während ihres Krankenhausaufenthaltes irgendwann auch ein Delir durchmachen (Lipowski, JAMA 258 (1987), 1789-1792).

Die EP-A-0 821 957 beschreibt die Verwendung von Xanomelin zur Behandlung von Entzugserscheinungen nach Absetzen von z.B. Alkohol, Pioiden, Cocain, Marijuana, Amphetaminen, Halluzinogenen etc., und zur Behandlung von Suchtdrogen-Mißbrauch im Allgemeinen und von Alkohol-Mißbrauch im Speziellen. Dabei wird auch das Symptom "Delirium (tremens)" z.B. als Folge von häufiger Alkohol-Intoxikation erwähnt.

Jedoch moduliert Xanomelin muscarinische cholinerge Rezeptoren, d.h. es wird als muskarinischer Agonist betrachtet.

Der EP-A-0 821 957 ist kein Hinweis zu entnehmen, der die Verwendung von Cholinesterase-Hemmern nahelegen würde.

Die WO-A-9 817 214 beschreibt die Verwendung des muskarinischen Agonisten Xanomelin zur Behandlung von HIV assoziierter Demenz, die sich auch symptomatisch als Delirium manifestieren kann.

Dabei handelt es sich um ein anticholinerges Delir, da ein Delir im Zuge einer HIV-Erkrankung mit strukturellen Veränderungen (Schädigungen) des cholinergen Systems aufgrund entzündlicher Reaktionen einhergeht. Derartige strukturelle Veränderungen sind mit degenerativen Prozessen des cholinergen Systems wie bei Alzheimer oder Parkinson vergleichbar.

Die Aufgabe der vorliegenden Erfindung besteht daher darin, eine Präparation zur Behandlung von Delir-Zuständen zur Verfügung zu stellen, die schnell und universell anwendbar ist, ohne daß es aufwendigster Ursachenforschung bedarf, so daß auch akute Verwirrtheitszustände rasch und verläßlich verbessert, geheilt oder verhindert werden können.

Diese Aufgabe wird gemäß der Erfindung gelöst, indem Acetylcholinesterase-Hemmer zur Herstellung einer Präparation zur Behandlung von Delirien, die nicht durch anticholinerge Intoxikation oder Degenerierung des cholinergen Systems bedingt sind, verwendet werden.

Die gemäß der Erfindung erhältlichen Präparationen steigern die Aktivität des zentralen, cholinergen Nervensystems durch Hemmung der Acetylcholin-Esterase.

Überraschenderweise können durch Behandlung mit den erfindungsgemäß erhältlichen Präparationen die akuten Verwirrtheitszustände schnell und effektiv bekämpft werden, obwohl sie keinen primären Zusammenhang mit nicht-anticholinergen Delirien zu haben scheinen. Währand also bislang ausschließlich bei Verdacht auf ein medikamentös induziertes anticholinerges Delir der dafür zugelassene Cholinesterase-Hemmer Physostigmin parenteral verabreicht worden ist (was zu einer gewöhnlich guten Aufhellung des anticholinergen Delirs und Verbesserung der Orientierung. des Patienten führt), war die Verabreichung derartiger Substanzen für nicht-anticholinerge Delirien bislang nicht in Erwägung gezogen worden, insbesondere weil bisher kein Zusammenhang der erfindungsgemäß behandelten Delirien mit dem cholinergen System vermutet worden war.

Die erfindungsgemäß verwendeten Acetylcholinesterase-Hemmer führen zu vermehrter Aktivität cholinerger Synapsen, indem weniger Acetylcholin abgebaut wird oder Acetylcholin langsamer abgebaut wird. die anticholinerge Wirkung auf diese Synapsen verstärkt wird (Acetylcholin-Rezeptorkanal-Aktivatoren) oder Autorezeptor-Agonisten des cholinergen Systems.

So können erfindungsgemäß auch Patienten nach langen Operationen mit Blutverlust, intraoperativer Hypoxie, präoperativ schlechtem kognitiven Status oder sehr hohem Lebensalter, also Patienten bei denen Risikofaktoren für das Auftreten eines postoperativen Delirs vorliegen nach ihrer Operation mit Aktivatoren des cholinergen Systems behandelt werden, um die Entwicklung des Delirs gar nicht erst zuzulassen (sekundäre Prävention des Delirs).

Mit der vorliegenden Erfindung konnte aber belegt werden, daß pathophysiologisch den meisten akuten Verwirrtheitszuständen wahrscheinlich eine funktionelle Imbalance verschiedener Transmittersysteme zugrundeliegt, wobei es wahrscheinlich zu einem relativen Überwiegen monaminerger Systeme kommt und das cholinerge System relativ unteraktiv ist.

Auf diesem Ansatz aufbauend kann daher jedes, nicht nur das anticholinerge Delir, durch eine cholinomimetische Therapie bekämpft, abgekürzt bzw. aufgehellt werden. Dies hat sich in einer Einzelfallbeobachtung eindrucksvoll bestätigt.

Die gemäß der Erfindung erhältlichen Präparationen können für die Therapie akuter Verwirrtheitszustände jeglicher Art und Ursache mittels pharmakologischer Aktivierung des cholinergen Systems z.B. durch die modernen Acetylcholin-Esterase-Hemmer wie Rivastigmin, Galanthamin, Tacrin oder Donepezil oder Folgepräparate mit direktem oder indirektem, cholinomimetischem Angriffspunkt verwendet werden. Während diese Therapie beim anticholinergen Delir - aufgrund der bisherigen Behandlungspraxis, die stets in die Ursachenbekämpfung wies - naheliegend ist, ist sie bei allen anderen Delirien eine völlig neue therapeutische Indikation dieser für die Therapie der Demenz vom Alzheimertyp entwickelten oder gerade in Entwicklung befindlichen Medikamente.

Die Definition des akuten Verwirrtheitszustandes oder Delirs war in ihrer Geschichte oft unscharf. So geht der Begriff der "organischen Psychose" auf K.Bonhoeffer zurück, der zwischen akuten "exogenen Reaktionstypen", also ätiologisch unspezifischen Reaktionen des Gehirns auf akute Schädigungen und chronischen exogenen Reaktionstypen unterschied. Letztere sind z.B. die Hirnleistungsschwäche, also die Störung von Gedächtnis und Denken, oder die Demenz, die damals nur bei schwerer irreversibler Hirnleistungsschwäche diagnostiziert wurde. Können so im weiteren Sinne also alle psychischen Störungen infolge von Hirnschädigungen "organische Psychosen" genannt werden, wird der Begriff "organische Psychose im engeren Sinn" häufig nur auf die akuten Psychosen bei Hirnschädigung angewandt und entspricht damit dem international unüblichen geschichtlichen Begriff des "akuten exogenen Reaktionstypus". Andere synonym verwendete Begriffe sind die "symptomatischen Psychosen", die "körperlich begründbaren Psychosen" oder der die Reversibilität dieser Zustandsbilder ausdrückende Begriff der "Funktionspsychosen". Dem letzten Begriff ähnlich ist auch der hoffnungsfrohe Begriff des "Durchgangssyndroms".

Heute werden nicht mehr diese Begriffe für die "organischen Psychosen im engeren Sinn" gebraucht, sondern es wird nur mehr die moderne international gebräuchliche diagnostische Entität "Akuter Verwirrtheitszustand (=Delirium)" verwendet, deren Umfang heute allgemein anerkannt und definiert ist.

So sind die oben beschriebenen "organischen Psychosen im engeren Sinn" in beiden heute verwendeten modernen psychiatrischen Diagnosesystemen, also dem DSM-IV (American Psychiatric Association: Diagnostic and Statistical Manual of Mental Disorders, Fourth Edition. Washington, D.C, APA, 1994) und dem ICD-10 (ICD-10,WHO, Internationale Klassifikation psychischer Störungen. Bern, H.Huber, 1991) so benannt und sehr ähnlich definiert worden. Heute spricht das Diagnosesystem der American Psychiatric Association APA, das Diagnostic and Statistic Manual DSM in seiner 4. Überarbeitung (DSM-IV, 1994) vom Delir und unterteilt in
(1) Delir auf Grund eines medizinischen Krankheitsfaktors, also z.B. postoperatives Delir, Delir nach hypoglykämischem Koma, Delir nach Reanimation, ...)
(2.1) Substanzinduziertes Delir, Unterform Substanzintoxikationsdelir, also z.B. Delir nach Intoxikation mit Alkohol, Amphetaminen, Opiaten, Atropin, etc. bzw.
(2.1) Substanzinduziertes Delir, Unterform Substanzentzugsdelir, also Delir im Alkoholentzug, Drogenentzug, Schlafmittelentzug, etc. und
(3) Delir aufgrund multipler Ätiologien. Bei unsicherer Ursache, wird
(4) ein "nicht näher bezeichnetes Delir" diagnostiziert.

Im Diagnosesystem der Weltgesundheitsorganisation WHO, dem ICD-10 (1991), wird unter den organischen psychischen Störungen neben den Demenzen, das "Delir, nicht durch Alkohol oder psychotrope Substanzen bedingt" definiert.

Da ein Delir bei jeder hirnorganischen Grundkrankheit wahrscheinlicher auftritt, gibt es immer wieder auch Patienten, die an einer Demenz leiden, welche augenblicklich durch ein Delir kompliziert ist. Hier diagnostiziert das DSM-IV zuerst die Demenz und mit Zusatzziffer ein zusätzlich vorhandenes Delir, während im ICD-10 in erster Linie das Delir als "Delir bei Demenz" diagnostiziert wird. Wichtig ist, daß die überwiegende Zahl der akuten Verwirrtheitszustände nicht an das Vorliegen einer Demenzerkrankung gebunden ist.

In der Klinik bezeichnen Delirien (akute Verwirrtheitszustände) eine gewöhnlich recht plötzlich auftretende und vorübergehende Störung intellektueller Funktionen mit immer vorhandener Störung der Aufmerksamkeit (qualitative und quantitative Bewußtseinsstörung) häufig begleitet von Störungen des Schlaf-Wach-Rhythmus und psychomotorischen Auffälligkeiten (hypoaktives oder hyperaktives Delir).

Wahrnehmung, Denken und Gedächtnisfunktionen sind bei akuten Verwirrtheitszuständen teilweise oder schwer gestört. Die Wahrnehmungsschärfe nimmt ab, es kommt zu Fehlinterpretationen von Wahrnehmungen, die für den Eindruck der Verwirrung verantwortlich sind. Neben illusionären Verkennungen kommen auch insbesondere optische Halluzinationen vor, die nicht nur einfache Inhalte, sondern auch sich bewegende Menschen, Tiere und ganze Szenarien umfassen. Bei plötzlichem Auftreten solcher Phänomene sind die Patienten gewöhnlich äußerst ängstlich, versuchen zu fliehen oder gegen die Erscheinungen zu kämpfen.

Das Denken ist desorganisiert, Denken und Sprache inkohärent, die Überstiegsfähigkeit geht verloren, abstrakte Konzepte können nicht mehr begriffen werden, die Fähigkeit des "inneren Vorstellens" (*e*: imagery) geht verloren. Die Patienten antworten daher zufällig und sinnlos, ohne diese Sinnlosigkeit selbst zu bemerken. Die Wahrnehmungs- und Denkstörung gemeinsam mit der immer gestörten Gedächtnisfunktion begünstigt das Auftreten von wahnhaften Interpretationen und Wahngewißheiten. Als Folge dieser intellektuellen Störung ist die Orientierung zu Zeit und Ort, gelegentlich auch zur Situation, selten zur Person, verlorengegangen.

Die Aufmerksamkeit ist immer gestört, was sich auch im pathologischen EEG nachvollziehen läßt. Die Patienten sind hochgradig ablenkbar, können ihre Aufmerksamkeit nicht willentlich modulieren oder neuen Inhalten zuwenden. Die Aufmerksamkeitsstörung zeigt sich z.B. in gestörtem Zahlen- oder Buchstabennachsprechen. Bei leichten akuten Verwirrtheitszuständen ist die Aufmerksamkeit stark schwankend, so daß einzelne Leistungen plötzlich sehr gut, Sekunden später wiederum überhaupt nicht erbracht werden können. Diese Schwankungen der Aufmerksamkeit sind in einigen Minuten dauernden Konzentrationstests (z.B. Durchstreichtests) gut beobachtbar. Die Störung der Aufmerksamkeit zeigt auch eine große Überlappung mit dem Begriff des gestörten Bewußtseins. Die qualitative Bewußtseinsstörung der Verwirrtheit kann jedoch auch mit einer nur sehr geringen quantitativen Bewußtseinsstörung (Müdigkeit) einhergehen.

Die Wachheit der Patienten ist untertags oft herabgesetzt, besonders typisch nicken die Patienten untertags immer wieder ein, während sie nachts leicht aufwachen bzw. überhaupt wach sind und dann auch besonders agitiert und ängstlich Unruhe verbreiten.'In schweren Fällen ist der Schlaf-Wach-Rhythmus vollkommen umgekehrt. Bei diesen Patienten ist eine erhöhte Aktivität und Unruhe besonders in den späten Nachmittags- und Abendstunden typisch.

Delirante Patienten können eine herabgesetzte oder gesteigerte Psychomotorik bieten, die sich in der Gestik und in der Sprache äußert. Während Patienten mit gesteigerter Psychomotorik vom Pflegepersonal und von Angehörigen rascher identifiziert werden, besteht die Gefahr, daß inaktive Patienten nicht als Verwirrtheitszustand diagnostiziert und behandelt werden.

Die Stimmung deliranter Patienten kann depressiv, häufig aber auch dysphorisch (grantig-reizbar) gefärbt sein, was mit der erhöhten Reagibilität auf Außenreize zu tun hat. Das sympathische Nervensystem ist übererregbar, es können vegetative Symptome wie starkes Schwitzen, Tachykardie, Schwankungen von Blutdruck und Puls, Gesichtsröte, weiter gestellte Pupillen, Angst und Wutreaktionen auftreten.

Mit den gemäß der Erfindung erhältlichen Präparationen können daher alle Delirien behandelt werden, die nicht anticholinerg sind, d.h., bei welchen das Delir nicht durch z.B. anticholinerge Intoxination (z.B. durch Tollkirschen-Vergiftung oder Vergiftung mit anderen Tropan-Alkaloiden) oder durch Degenerierung des cholinergen Systems bei degenerativer Demenz bedingt ist (vgl. Int. Psychoger. 3(2) (1991), 373-395; Psych. Clin. Neuroscience 248 (Supp. 1) (1998), S. 59).

Bevorzugterweise wird mit den erfindungsgemäß erhältlichen Präparationen ein postoperatives Delir, ein Delir während interner Erkrankung oder ein posttraumatisches Delir behandelt. Besonders bei diesen klinisch äußerst wichtigen Delirien steht bislang keine befriedigende medikamentöse Therapie zur Verfügung.

Eine weitere bevorzugte Ausführungsform der vorliegenden Erfindung betrifft die Verwendung der erfindungsgemäß erhältlichen Präparationen zur Behandlung von Delirien, die durch nicht-anticholinerge Intoxikationen bedingt

Ein weiteres Einsatzgebiet der Erfindung liegt in der Behandlung von Substanzentzug-Delirien, bei welchen ebenfalls ein ganz besonders großer Bedarf an effektiven Behandlungsverfahren herrscht.

Weiters kann erfindungsgemäß auch ein Delir, welches durch hypoglykämische Prozesse, insbesondere hypoglykämisches Koma, bedingt ist, gut behandelt werden.

Häufig treten Delirien auch in Zusammenhang mit Reanimation auf, welche ebenfalls mit den erfindungsgemäß erhältlichen Präparationen behandelt werden können.

Allen angeführten Delirien ist gemein, daß sie keinen vordergründigen Zusammenhang mit dem cholinergen System zu haben scheinen, wodurch deren effektive Behandlung mit den erfindungsgemäßen Maßnahmen besonders überraschend ist.

Die Differentialdiagnose der akuten Verwirrtheitszustände (des Delirs) ist aus der Psychopathologie im Querschnitt und der Information über den Verlauf aus der Fremdanamnese möglich. Einen spezifischen diagnostischen Test gibt es nicht, wenn auch ein Delir bei unauffälligem EEG ausgeschlossen werden kann. Neben einer Demenz muß bei Diagnose eines Delirs auch eine Schizophrenie, eine Manie und ein psychogener Dämmerzustand ausgeschlossen werden. Darüber hinaus werden auch immer wieder Aphasien nach Schlaganfällen besonders bei älteren Menschen mit Delirs verwechselt. Schizophrene Exazerbationen, auch beim älteren Menschen, gehen eher mit auditorischen als mit visuellen Halluzinationen einher. Darüber hinaus ist in diesen delirant anmutenden Zuständen bei endogenen Psychosen das EEG in der Regel unauffällig.

Die wesentliche Unterscheidung zwischen dem reinen Delir und einer Demenzerkrankung bringt die Anamnese, wobei das Delir akut beginnt und oft nachts erstmals von Angehörigen oder Pflegepersonal beobachtet wird, während die Demenz schleichend beginnt und im Tagesablauf entdeckt wird. Während der Zustand des dementen Patienten über Tage und Wochen relativ stabil ist, kommt es beim Delir zu starken Fluktuationen auch innerhalb des Tages und häufigen kurzen Episoden guter Orientierung. Die Aufmerksamkeit ist bei beginnenden Demenzen in der Regel nicht herabgesetzt bzw. wirken diese Patienten sehr bemüht und wach, wohingegen delirante Patienten sehr leicht ablenkbar sind und damit unkonzentriert wirken.

Es gibt keine repräsentative epidemiologische Untersuchung zur Häufigkeit akuter Verwirrtheitszustände, jedoch weisen Untersuchungen an auf internen Stationen aufgenommenen Patienten darauf hin, daß die akuten Verwirrtheitszustände eine größere Inzidenz als selbst dementielle und depressive Syndrome bei alten Menschen haben. Akute Verwirrtheitszustände werden bei 30 bis 50 % der Patienten über 70 Jahre zu irgendeinem Zeitpunkt während einer internen Aufnahme beobachtet.

Die Behandlung mit den erfindungsgemäß erhältlichen Präparationen erfolgt durch Verabreichung einer geeigneten Dosis eines Mittels, welches direkt oder indirekt die Aktivität des (zentralen) cholinergen Nervensystems zu steigern vermag, an einen Patienten. Da eine große Anzahl derartiger Medikamente bereits für andere Indikationen am Markt ist, kann die Dosis für die erfindungsgemäße Behandlung auf Grundlage der bereits angewendeten Dosierungen ermittelt werden. Aufgrund der guten Wirksamkeit der erfindungsgemäßen Behandlung ist die Gabe von Neuroleptika erfindungsgemäß nicht mehr erforderlich.

Begleitende Behandlungsmaßnahmen sind jedoch wie bei der bisherigen Behandlungspraxis sinnvoll: So sollte der Elektrolyt- und Flüssigkeitshaushalt kontrolliert werden und die Ernährung und Versorgung mit den notwendigen Vitaminen und Spurenelementen sichergestellt werden.

Ein in Realitätsorientierung trainiertes Pflegepersonal beschleunigt die Reorientierung deliranter älterer Menschen nachweislich. Die Patienten sollten in ruhigen, gut beleuchteten Räumen verweilen, dort auch gewohnte Objekte um sich haben und auch eine Uhr und einen Kalender sowie Tageszeitungen zur Verfügung haben. Die Patienten sollten immer wieder auf ihren Orientierungssinn angesprochen und verbal reorientiert werden.

Ein Delir ist per definitionem eine vorübergehende Erkrankung, die in der Regel nur einige Tage bis zu wenigen Wochen dauert. Aber ein Delir ist wegen der begleitenden massiven vegetativen Symotomatik beim multimorbiden älteren Patienten eine gefährliche Krankheitskomplikation. Nicht nur kommt es über die Belastung des Herzkreislauf-Systems zu einer verschlechterten kardialen Situation, sondern es sind im Zustand der Verwirrtheit auch Selbstbeschädigungen und Verletzungen möglich. Darüber hinaus entfernen sich delirante Patienten die intravenösen Katheter, öffnen sich Verbände und stürzen besonders leicht nachts aus dem Bett. Werden die Patienten zu stark sediert, drohen tiefe Beinvenenthrombosen und Pulmonalembolien. Insgesamt ist die Mortalität des Delirs wissenschaftlich schwer abzuschätzen, weil die deliranten Zustände auch bei ernsten extracerebralen Erkrankungen begleitend auftreten und die Mortalität dieser Erkrankungen dann nicht von der Mortalität des Delirs getrennt werden kann.

Ergebnisse aus der Arbeitsgruppe von Levkoff (Arch. Intern. Med. 152 (1992), 334-40) legen nahe, daß zahlreiche Patienten nach Erleiden eines akuten Verwirrtheitszustandes wiederum solche Zustände erleiden können, die völlige Restitutio ad integrum also nicht bei allen Patienten wie erhofft eintritt. Diese Persistenz der Symptome ist gegenwärtig Inhalt internationaler Forschung und in ihrer Pathophysiologie unklar.

Erstaunlicherweise gibt es zur Zeit keine international anerkannte Theorie zu den cerebralen Entstehungsbedingungen eines deliranten Syndroms. Insbesondere ist auch die Frage, ob es sich bei einem Delir um die Folge einer diffusen Hirnfunktionsstörung oder um eine lokalisierte Störung, z.B. in unserem Aufmerksamkeitssystem handelt, unbeantwortet. Beeinflußt durch die leichte Auslösbarkeit eines Delirs durch anticholinerg wirkende Substanzen gibt es auch eine biochemische Theorie der Imbalance zentral cholinerger und monaminerger (noradrenerger und/oder serotonerger und/oder dopaminerger) Mechanismen als Grundlage des Delirs. Wahrscheinlich liegen den zahlreichen deliranten Syndromen verschiedene pathogenetische Mechanismen in teils gut lokalisierbaren Systemen, teils als Folge diffuser cerebraler Störungen zugrunde. Während delirante Syndrome nach Hypoglykämien, nach Herzrhythmusstörungen bzw. Blutdruckabfällen sicherlich Ausdruck von Funktionsstörungen von Nervenzellen und Neuronensystemen in weiten Teilen des Großhirns darstellen, sind andere delirante Syndrome, wie z.B. die Korsakoff-Psychose, Ausdruck von umschriebenen Läsionen im Stammhirn und Zwischenhirn.

Einen ganz anderen Zugang zur Pathogenese der Verwirrtheitszustände liefert der Begriff der Homöostase. Es ist ein gerontologisches Prinzip, daß eine wichtige Auswirkung der Alterung eines Lebewesens seine fortschreitend geringer werdende Anpassungsfähigkeit an Veränderungen der Umwelt darstellt, wie sie eben im Falle des Gehirns durch Medikamente, Hypoglykämien und fokale Läsionen zustande kommen. Strukturen und Funktionen von Teilen des Organismus besitzen in unterschiedlichen Lebensphasen unterschiedliche Flexibilität, innerhalb derer bei Belastungen der Normbereich des Funktionierens eingehalten werden kann. Diese Anpassungsfähigkeit ist das Resultat einer Vielzahl von Einzelreaktionen z.B. auf Synapsenniveau, die von der Wissenschaft gewöhnlich isoliert analysiert werden. Nervenzellen, Neuronensysteme, das Gehirn und unser Körper befinden sich zu jeder Zeit in einem dynamischen und wechselseitig voneinander abhängigen Zustand, der als Homöostase bezeichnet wird und ein Steady-State-Gleichgewicht ist, das sich nur innerhalb eines gewissen Bereichs auf Störungen einstellen kann. Ein derartiges Fließgleichgewicht garantiert Kontinuität, gibt aber auch die Möglichkeit zur Anpassung. Prozesse, die der Aufrechterhaltung der Homöostase dienen, sind Systeme der Adaptivität oder Kompensation.

Mit zunehmendem Alter wird die Kontrolle der Homöostase schwieriger, die Bandbreite des Normbereichs enger, die Stabilität und Kapazität der Kompensationsfähigkeit geringer. Dies drückt sich in einer wachsenden Störanfälligkeit aus, die bei den Funktionen des Bewußtseins, der Vigilanz, der Aufmerksamkeit oder der Orientierung das Delir bedeutet. Ein recht gut untersuchtes Beispiel einer im Alter weniger adaptierbaren zentralen Funktion ist die Thermoregulation, an deren Beispiel sich die altersabhängige Einschränkung der Anpassungsfähigkeit einer Gehirnfunktion zeigen ließ. Folge dieser gestörten Adaptivität ist auch die mit zunehmendem Lebensalter deutlich gesteigerte Empfindlichkeit auf Psychopharmaka.

Eine im zeitlichen Rahmen nicht mehr zu kompensierende Ausschwankung des Gleichgewichts verschiedener Transmittersysteme unseres Gehirns, sei es durch Psychopharmaka oder andere Störeinflüsse, führt zum Zusammenbruch der höchsten Ebene der cerebralen Homöostase, des Verarbeitens bewußter Denkinhalte. Folglich treten Delirien bei langsam wirkenden Störungen von Funktion oder Struktur des Gehirns nicht auf, sondern werden bei sehr plötzlichen Veränderungen des Hirnfunktionsniveaus eher beobachtet.

Wie aus obigen Ausführungen hervorgeht, können unzählige Ursachen einem Delir zugrunde liegen. Klinisch wichtig ist die Unterscheidung von primär cerebralen und sekundär cerebralen Ursachen und das rasche Erkennen gut behandelbarer Ursachen wie Medikamentennebenwirkungen, Störungen des Wasser- und Elektrolythaushalts, Vitaminmangelzustände und kardiovaskuläre Störungen. Der bedeutendste Risikofaktor für das Auftreten eines Delirs ist das Lebensalter. Die folgende Tabelle zeigt eine Übersicht der wichtigsten Ursachen akuter Verwirrtheit.

### Tabelle: Die wichtigsten Ursachen akuter Verwirrtheitszustände

1. primär cerebral:
   Schädel-Hirn-Trauma
   primär degenerative Erkrankungen
   raumfordernde Prozesse
   entzündliche Prozesse
   vaskulär-ischämische Prozesse
   Intoxikationen (nicht nur anticholinerge)
   0₂-Mangel (Anämie, respiratorische Insuffizienz)
2. sekundär cerebral:
   Postoperativ
   Nach Anästhesie (auch ohne Operation)
   Intoxikationen
   Medikamentennebenwirkungen
   kardiovaskuläre Störungen
   metabolisch-toxisch (Niere, Leber, ..)
   Wasser- und Elekrolythaushalt
   endokrine Störungen (Schilddrüse, Nebenschilddrüse)
   Mangel an Vitaminen oder Spurenelementen
   Infektionen
   Thermoregulationsstörungen
   Störungen der Sinnesorgane
   Streß

Das postoperative Delir ist einer der Hauptanwendungsbereiche der erfindungsgemäßen Therapie und soll deshalb kurz extra beschrieben werden. Bedingt durch die Zunahme an aufwendigen Operationen im Bereich der Herz- und Thoraxchirurgie, der Neurochirurgie, der Transplantationschirurgie aber auch der Unfallchirurgie, Bauchchirurgie etc. treten trotz Verbesserungen der Operations- und Narkosetechnik immer mehr postoperative Verwirrtheitszustände auf. Einerseits vermindern die besseren Techniken zwar das Auftreten dieser gefürchteten Operations-Spätkomplikation, andererseits werden wegen der verbesserten Techniken immer schwerer kranke, multimorbide und auch immer ältere Menschen diesen Eingriffen unterzogen. Trotz der großen klinischen Bedeutung sind diese Verwirrtheitszustände wenig beforscht, da diese psychiatrische Krankheit nur ganz selten in psychiatrischen Krankenhäusern behandelt wird. Die Inzidenz des postoperativen Deliriums schwankt je nach Studie und untersuchter Patientenpopulation bzw. Operation zwischen 0 % und 73,5 % (Dyer et al., Postoperative Delirium. Archives of Internal Medicine, 155 (13)(1995), 461-465). Das Auftreten eines postoperativen Deliriums ist ein Risikofaktor für eine erhöhte Komplikationsrate und erhöhte Mortalität, erhöht die Wahrscheinlichkeit der Pflegeheimeinweisung, verlängert die Krankhausaufenthaltsdauer, es folgen mehr und damit teurere Wiederaufnahmen und der postoperative Verwirrtheitszustand kann langdauernde geistige Folgen haben.

Ein Beispiel für die Häufigkeit der postoperativen Verwirrtheitszustände kann die Orthopädie geben. In den 3 weltweit bislang durchgeführten prospektiven Studien erlitten zwischen 18 und 41 % der Patienten, die geplant einer Hüfttransplantation unterzogen wurden, diese Komplikation. In den 4 prospektiven Studien an Patienten mit Hüfttransplantation nach Schenkelhalsfraktur, also bei ungeplantem Eingriff nach Trauma, liegen die Häufigkeiten dieser psychiatrischen Komplikation bei 26 % bis 52 % der Patienten!

Die Erfindung wird anhand des folgenden Beispiels, auf das sie selbstverständlich nicht beschränkt ist, näher erläutert.

**Beispiel:** Behandlung eines nicht-anticholinergen Delirs mit dem oral verabreichbaren modernen Hemmer der Acetylcholin-Esterase Rivastigmin

Eine 70-jährige Patientin leidet seit ihrem 35. Lebensjahr an einer manisch-depressiven Krankheit. Bis zuletzt bestanden keinerlei geistige Abbauerscheinungen. Nach einem Harnwegsinfekt bei Lithiumtherapie der manisch-depressiven Krankheit kommt es zu einer Lithiumintoxikation mit schwerem, hyperaktivem Delir, das zur stationären Aufnahme auf einer internen Station zwingt. Derartige Lithiumintoxikations-Deliria klingen in der Regel erst nach 6 - 8 Wochen vollständig ab. Die Patientin wird nach 4 Tagen in die Privatklinik überstellt und ist hochpsychotisch, uriniert in die Ecke des Zimmers, spricht mit der Nachttischlampe, halluziniert fremde Personen und Zwerge im Zimmer, flieht wiederholt die Station, glaubt das Pflegepersonal will sie töten, ist motorisch unheimlich unruhig und getrieben, nestelt an Bettdecke und Telefonkabel, schläft untertags immer wieder ein, ist jedoch die ganze Nacht auf. Die Patientin weint und lacht abwechselnd, fürchtet sich immer wieder plötzlich, um dann wieder zu lachen. Alle diese Symptome sind sehr instabil, stark wechselnd, die Patientin jedoch am Aufnahmetag nie wirklich ansprechbar. In der Delirium Skala nach Trzepacz (Psychiatry Research, 23, (1988), 89-97) wird ein Score von 29 (von 32 erreichbaren Punkten) entsprechend einem schweren Delir erreicht. Nach 48 Stunden Therapie mit Exelon® (Rivastigmin) in der Dosis von 2x1,5 mg ist die Patientin orientiert, halluziniert nicht mehr, erinnert sich zwar an ihre Wahnideen, distanziert sich jedoch von ihnen, ist noch leicht unruhig, leicht unkonzentriert, ist untertags noch schläfrig, jedoch besteht nächtens nur mehr diskrete Durchschlafstörung. Die Symptome sind nun stabil ohne besondere Schwankungen, der Deliriumscore sinkt innerhalb von 48 Stunden auf 8 Punkte. Nach 4 Wochen wird Rivastigmin (Exelon) abgesetzt und sogar jetzt noch treten für 2-3 Wochen Aufmerksamkeitsschwankungen, Schlafstörungen, Unruhe und Tagesschwankungen auf, wie sie nach dieser Zeit im Spontanverlauf der Lithiumintoxikation zu erwarten gewesen wären.

## Patentansprüche

1. Verwendung von Acetylcholinesterase-Hemmern zur Herstellung einer Präparation zur Behandlung von Delirien, die nicht durch anticholinerge Intoxikation oder Degenerierung des cholinergen Systems bedingt sind.

2. Verwendung nach Anspruch 1, **dadurch gekennzeichnet, dass** das Delir ein postoperatives Delir oder ein Delir während einer internen Erkrankung ist.

3. Verwendung nach Anspruch 1, **dadurch gekennzeichnet, dass** das Delir durch nicht-anticholinerge Intoxikationen bedingt ist.

4. Verwendung nach Anspruch 1, **dadurch gekennzeichnet, dass** das Delir durch Substanzentzug bedingt ist.

5. Verwendung nach Anspruch 1, **dadurch gekennzeichnet, dass** das Delir durch hypoglykämische Prozesse, insbesondere hypoglykämisches Koma, bedingt ist.

6. Verwendung nach Anspruch 1, **dadurch gekennzeichnet, dass** das Delir durch Reanimation bedingt ist.

## Claims

1. Use of acetylcholine esterase inhibitors for producing a preparation for the treatment of deliria not caused by anticholinergic intoxication or by degeneration of the cholinergic system.

2. Use according to claim 1, **characterised in that** the delirium is a post-operative delirium or a delirium during an internal disease.

3. Use according to claim 1, **characterised in that** the delirium is conditioned by non-anticholinergic intoxications.

4. Use according to claim 1, **characterised in that** the delirium is conditioned by substance withdrawal.

5. Use according to claim 1, **characterised in that** the delirium is conditioned by hypoglycaemic processes, in particular hypoglycaemic coma.

6. Use according to claim 1, **characterised in that** the delirium is conditioned by resuscitation.

## Revendications

1. Utilisation d'inhibiteurs de cholinestérase pour fabriquer une préparation de traitement de délires qui ne sont pas conditionnés par une intoxication anti-cholinergique ou une dégénérescence du système cholinergique.

2. Utilisation selon la revendication 1, **caractérisée en ce que** le délire est un délire postopératoire ou un délire au cours d'une maladie interne.

3. Utilisation selon la revendication 1, **caractérisée en ce que** le délire est conditionné par des intoxications non anticholinergiques.

4. Utilisation selon la revendication 1, **caractérisée en ce que** le délire est conditionné par le sevrage d'une substance.

5. Utilisation selon la revendication 1, **caractérisée en ce que** le délire est conditionné par des processus hypoglycémiques, en particulier, par le coma hypoglycémique.

6. Utilisation selon la revendication 1, **caractérisée en ce que** le délire est conditionné par une réanimation.
